# EUROPEAN PATENT APPLICATION

(11) **EP 3 251 667 A1**
(43) Date of publication of application: **06.12.2017**
(21) Application number: 16171943.0
(22) Date of filing: 30.05.2016
(51) Int. Cl.: A61K 31/202, A23L 29/00, A23L 33/00, A23L 33/12, A23K 20/158, A23K 10/22

(54) **NUTRITIONAL COMPOSITION**

(71) Applicant: Instituto Nacional de Medicina Genomica (INMEGEN), 14610 Mexico (MX)
(72) Inventor: TEJERO BARRERA, Elizabeth Maria, 03020 MEXICO (MX); BINIA, Aristea, 1700 Fribourg (CH); SILVA ZOLEZZI, Irma, 1084 Carrouge (CH); KUSSMANN, Martin, 1066 Epalinges (CH)
(74) Representative: Couzens, Patrick John

(57) **Abstract**

The present invention provides a nutritional composition comprising an omega-3 polyunsaturated fatty acid (PUFA) as an active agent for use in reducing long-term glucose levels in a subject.

## Description

### FIELD OF THE INVENTION

The present invention relates to nutritional compositions and methods useful for reducing long-term glucose levels in a subject.

### BACKGROUND TO THE INVENTION

Blood glucose level is the amount of glucose present in the blood of a human or animal. The body naturally tightly regulates blood glucose levels as a part of metabolic homeostasis.

Glucose is the primary source of energy for the body. It is transported from the intestines or liver to cells via the bloodstream, and is made available for cell absorption via the action of insulin, which is produced by β cells in the pancreas.

If blood sugar levels are too high the body suppresses appetite over the short term. However, long-term hyperglycemia causes many health problems including heart disease, eye, kidney, and nerve damage.

The association between diet, insulin levels and glucose levels has encouraged investigations into how various dietary interventions affect glucose levels. One intervention which has been investigated is omega-3 polyunsaturated fatty acids (omega-3 PUFA). However, the outcomes of these studies have been inconsistent and inconclusive.

By way of example, Ahren et al. (European Journal of Clinical Nutrition; 2009; 63; 778-786) reported that a 12-week supplementation with omega-3 PUFA plus conjugated linoleic acid (CLA) resulted in no significant difference in fasting glucose, insulin or C-peptide in treatment group compared to control group. Further, insulin secretion/sensitivity was not affected by supplementation in all young subjects and old lean subjects, whilst insulin sensitivity was decreased in old obese subjects.

Ramel et al. (Diabetologia; 2008; 51; 1261-1268) reported reduced fasting levels of insulin, reduced fasting glucose and improved insulin resistance following supplementation of young, overweight/obese subjects (BMI 27.5-32.5) with 1.3 g/day LC-PUFA. Lopez-Alarcon et al. (Archives of Medical Research; 2011; 42; 502-508) reported a reduction in fasting insulin levels in obese, insulin resistant children. However, there was no decrease in fasting glucose levels.

A number of the studies described above report fasting insulin and fasting glucose levels. Fasting glucose level tests are somewhat limited as they only provide information on the current blood glucose levels at the time the test is taken. As such, fasting glucose levels do not indicate the subject's blood glucose levels over a prolonged time period and the results of a fasting glucose test do not necessarily correlate with long-term glucose levels. In particular, a fasting glucose test may not identify an issue with long-term glucose levels or may be impacted by short-term fluctuations in blood sugar levels which are not clinically relevant.

There remains a need for methods for reducing long-term glucose levels *in vivo.*

### SUMMARY OF THE INVENTION

The present inventors have surprisingly determined that supplementation with omega-3 PUFA causes a reduction in long-term glucose levels. Furthermore, this reduction is seen in a broad spectrum of subjects and the effect may be independent of body mass index (BMI).

Thus, in a first aspect the present invention provides an omega-3 PUFA or a nutritional composition comprising an omega-3 PUFA as an active agent for use in reducing long-term glucose levels in a subject.

The subject may suffer from a condition associated with elevated long-term glucose levels.

In another aspect, the present invention provides an omega-3 PUFA or a nutritional composition comprising an omega-3 PUFA as an active agent for use in reducing long-term glucose levels to treat or prevent a condition associated with elevated long-term glucose levels in a subject.

The condition associated with elevated long-term glucose levels may be insulin resistance, prediabetes, type 2 diabetes, metabolic syndrome or cardiovascular disease.

The reduction in long-term glucose levels may be determined by reduced HbA1c levels.

The subject may be obese or non-obese. In one embodiment the subject is non-obese.

In one embodiment the subject has a BMI of 25 or less.

The subject may be administered for example, from about 500 to about 10000 mg omega-3 PUFA per day, preferably from about 1500 to about 3000 mg omega-3 PUFA per day.

In one embodiment the omega-3 PUFA comprises eicosapentanoic acid (EPA) and/or docosahexanoic acid (DHA).

The omega-3 PUFA may comprise an EPA to DHA ratio from about 0.5:1 to about 5:1, for example about 0.5:1, 1:1, 1.5:1, 2:1, 2.5:1, 3:1 or 5:1 wt/wt; preferably about 2.5:1 wt/wt.

The subject may be administered, for example from about 500 to about 4000 mg EPA and from about 500 to about 4000 mg DHA per day, preferably from about 1000 to about 3000 mg EPA and from about 500 to about 1000 mg DHA per day.

The nutritional composition may be in the form of a food stuff, preferably a human food stuff. The nutritional composition may be in the form of a complete nutritional product. The nutritional composition may be in the form of a capsule, a tablet, a powdered form, a freeze-dried product or an oil-in-water emulsion.

In one aspect the present invention provides an omega-3 PUFA or a nutritional composition comprising an omega-3 PUFA as an active agent for use in reducing Hb1Ac levels.

In a further aspect the present invention relates to a method for reducing long-term glucose levels in a subject comprising administering a nutritional composition comprising omega-3 PUFA as an active agent to the subject.

In a further aspect the present invention relates to a method for reducing long-term glucose levels to treat or prevent a condition associated with elevated long-term glucose levels in a subject comprising administering an omega-3 PUFA or a nutritional composition comprising omega-3 PUFA as an active agent to the subject.

In a further aspect the present invention relates to the use of an omega-3 PUFA in the preparation of a nutritional composition for reducing long-term glucose levels in a subject.

### DESCRIPTION OF THE FIGURES

**Figure 1** - Fasting Hb1Ac% levels before and after a six week supplementation with omega-3 PUFA (a) 2.7 g/day omega-3 PUFA (b) 1.8 g/day omega-3 PUFA
**Figure 2** - Stratification of fasting Hb1Ac% levels based on baseline Hb1Ac % - results are shown before and after a six week supplementation with omega-3 PUFA

### DETAILED DESCRIPTION OF THE INVENTION

### LONG-TERM GLUCOSE LEVELS

The nutritional compositions comprising an omega-3 PUFA described herein are used to reduce long-term glucose levels in a subject.

Blood glucose level is the amount of glucose present in the blood of a human or animal. The body naturally regulates blood glucose levels tightly as a part of metabolic homeostasis.

Glucose is the primary source of energy for the body. It is transported from the intestines or liver to cells via the bloodstream, and is made available for cell absorption via the action of insulin, produced by β cells in the pancreas.

If blood sugar levels are too high the body suppresses appetite over the short term. However, long-term high blood sugar levels (hyperglycemia) causes many health problems including heart disease, eye, kidney, and nerve damage.

Blood glucose levels within a given range are typically associated with good health. Hyperglycemia may be associated with conditions such as insulin resistance, prediabetes, type 2 diabetes, metabolic syndrome or cardiovascular disease. In contrast, low blood sugar levels may lead to potentially fatal hypoglycemia. Hypoglycemia may be associated with symptoms such as lethargy, impaired mental functioning; irritability; shaking, twitching, weakness in arm and leg muscles; pale complexion; sweating; paranoid or aggressive mentality and loss of consciousness.

Glucose levels are traditionally determined using the fasting plasma glucose (FPG) test or oral glucose tolerance test (OGTT). These tests may be limited because they only provide information on the current blood glucose levels at the time the test is taken. As such, fasting glucose levels may not accurately reflect a subject's blood glucose levels over a prolonged time period and the results of a fasting glucose test do not necessarily correlate with long-term glucose levels. In particular, a fasting glucose test may not identify an issue with long-term glucose levels or may be impacted by short-term fluctuations in blood sugar levels which are not clinically relevant.

Accordingly, long-term blood sugar levels may provide a more reliable measure of an individual's typical blood sugar level.

"Long-term glucose levels" may refer to the blood glucose level in a subject over a period of about 4 to about 16 weeks, about 4 to about 12 weeks or about 8 to about 12 weeks preceding a blood glucose test.

In a preferred embodiment, long-term glucose levels are determined using a glycohaemoglobin (haemoglobin A1c or Hb1Ac) test.

In one embodiment, by reduction in long-term glucose levels in a subject it is meant that Hb1Ac levels are reduced in said subject.

Glycated haemoglobin (Hb1Ac) is formed by the nonenzymatic attachment of glucose to the N-terminal valine of the β-chin of hemoglobin (Hb). The life span of erythrocytes is approximately 120 days, and consequently HbA1c levels reflect long-term glycemic exposure, representing the average glucose concentration over the preceding 8 to 12 weeks. The Hb1Ac test result does not change with any recent changes in diet, exercise, or medicines. As such the Hb1Ac test provides information on the long-term blood glucose levels of a subject.

A variety of Hb1Ac tests are known in the art, and these assays may be standardized according to the Reference Measurement Procedure (RMP) of the International Federation of Clinical Chemistry (IFCC) (Jeppsson et al.; Clin Chem Lab Med. 2002;40:78-89 & Hoelzel et al.; Clin Chem. 2004;50:166-174).

Analytical methods for determining Hb1Ac levels include:
- cation exchange chromatography (where Hb1Ac and Hb are separated by a difference in their isoelectric points),
- affinity chromatography (which typically utilizes m-aminophenyl boronic acid and depends on a specific interaction between the glucose on Hb1Ac and the immobilized boronic acid),
- capillary electrophoresis (which separates Hb1Ac and Hb according to their electrophoretic mobility in an alkaline buffer with a specific pH), and
- immunoassay (typically using an antibody which specifically binds the β N-terminal glycated tetrapeptide or hexapeptide group of Hb1Ac).

Long-term glucose levels determined using Hb1Ac tests are typically expressed as SI units (i.e., HbA1c/total Hb (mmol/mol)) and/or derived NGSP/DCCT units (%). The master equations for converting IFCC units into NGSP units (NGSP%=0.0915×IFCC mmol/mol+2.15) and vice versa (IFCC mmol/mol=10.93 NGSP%-23.5) are established and monitored by the IFCC and NGSP networks (Weykamp et al.; Clin Chem. 2008;54:240-248).

The interpretation of each of these units with respect to diabetes and the risk of developing diabetes are shown in Table 3.

**Table 3**

| **Standard interpretation norm*** | | **IFCC (mmol/mol)** | **NGSP (%)** |
|---|---|---|---|
| Normal reference range | | 20-42 | 4-6 |
| Diagnosis | Low risk | <40 | <5.8 |
| | Increasing risk future diabetes | 40-46 | 5.8-6.4 |
| | Diabetes | >46 | >6.4 |

| | | | |
|---|---|---|---|
| *Diabetes Control and Complications Trial (DCCT) 1993; American Diabetes Association (ADA) 2010; ADA 2011. | | | |

The term "reducing" is synonymous with terms such as decreasing and lowering. The term "levels" refers to any measure of abundance such as amount or concentration. Thus, the present compositions and methods are useful for decreasing long-term glucose levels in a subject.
Reducing long-term glucose levels may mean that long-term glucose levels are reduced by at least 0.5%, at least 1%, at least 2%, at least 3%, at least 5%, at least 7%, at least 10%, at least 15%, at least 20%, at least 25%,at least 30%, at least 40%, at least 50%, at least 60% or at least 70%, compared to the long-term glucose level prior to the administration of a nutritional composition comprising omega-3 PUFA as described herein.

In one embodiment, long-term glucose levels are determined using an Hb1Ac test and Hb1Ac levels are reduced by at least 0.5%, at least 1%, at least 2%, at least 3%, at least 5%, at least 7%, at least 10%, at least 15%, at least 20%, at least 25%, or at least 30% or at least 40% compared to Hb1Ac levels prior to the administration of a nutritional composition comprising an omega-3 PUFA as described herein.

Long-term glucose levels, as determined using an Hb1Ac test for example, are typically determined using blood serum or plasma. Techniques for collecting blood samples and separating blood fractions are well known in the art. For instance, vena blood samples can be collected from patients using a needle and deposited into plastic tubes. The collection tubes may, for example, contain spray-coated silica and a polymer gel for serum separation. Serum can be separated by centrifugation at 1300 RCF for 10 min at room temperature and stored in small plastic tubes at -80°C.

In one embodiment, the present invention relates to an omega-3 PUFA or a nutritional composition as described herein for use in reducing long-term glucose levels in a subject suffering from a condition associated with elevated long-term glucose levels. Such conditions include, for example, insulin resistance, prediabetes, type 2 diabetes, metabolic syndrome or cardiovascular disease.

In one embodiment, the present invention relates to the use of an omega-3 PUFA or a nutritional composition as described herein for use to treat or prevent a condition associated with elevated long-term glucose levels in a subject.

The prevention of a condition associated with elevated long-term glucose levels relates to the prophylactic use of an omega-3 PUFA or a nutritional composition as described herein. Accordingly, the nutritional composition may be administered to a subject who has not yet developed a condition and/or who is not showing any symptoms of the condition to prevent or impair the cause of condition or to reduce or prevent development of at least one symptom associated with the condition.

Insulin resistance is a condition in which the body produces insulin but does not use it effectively. When people have insulin resistance, glucose builds up in the blood instead of being absorbed by the cells, leading to type 2 diabetes or prediabetes. Insulin resistance may be defined as a reduced responsiveness of a target cell or a whole organism to the insulin concentration to which it is exposed. This definition is generally used to refer to impaired sensitivity to insulin mediated glucose disposal.

Prediabetes is the medical stage in which not all of the symptoms required to diagnose a person as diabetic are present, but blood sugar is abnormally high. Prediabetes usually occurs in people who already have insulin resistance. Although insulin resistance alone does not cause type 2 diabetes, it often sets the stage for the disease by placing a high demand on the insulin-producing beta cells. In prediabetes, the beta cells can no longer produce enough insulin to overcome insulin resistance, causing blood glucose levels to rise above the normal range

Type 2 diabetes is a chronic metabolic disorder which is increasing in prevalence globally. In some countries of the world the number of people affected is expected to double in the next decade due to an increase in the ageing population.

Type 2 diabetes is characterized by insulin insensitivity as a result of insulin resistance, declining insulin production, and eventual pancreatic beta-cell failure. This leads to a decrease in glucose transport into the liver, muscle cells, and fat cells. There is an increase in the breakdown of fat associated with hyperglycemia.

As a result of this dysfunction, glucagon and hepatic glucose levels that rise during fasting are not suppressed with a meal. Given inadequate levels of insulin and increased insulin resistance, hyperglycemia results.

People with type 2 diabetes are more vulnerable to various forms of both short- and long-term complications, including diabetic ketoacidosis (DKA), hyperosmolar hyperglycaemic state (HHS), retinopathy, cardiopathy, nephropathy and neuropathy. These complications may lead to premature death.

Metabolic syndrome is a clustering of at least three of five of the following medical conditions: abdominal (central) obesity, elevated blood pressure, elevated fasting plasma glucose, high serum triglycerides, and low high-density lipoprotein (HDL) levels. Metabolic syndrome is associated with the risk of developing cardiovascular disease and diabetes

Cardiovascular disease (CVD) is a class of diseases that involve the heart or blood vessels. Cardiovascular disease includes coronary artery diseases (CAD) such as angina and myocardial infarction (commonly known as a heart attack). Other CVDs are stroke, hypertensive heart disease, rheumatic heart disease, cardiomyopathy, atrial fibrillation, congenital heart disease, endocarditis, aortic aneurysms, peripheral artery disease and venous thrombosis.

### BMI

"Body mass index" or "BMI" means the ratio of weight in kg divided by the height in metres, squared.

"Overweight" may be defined for an adult human as having a BMI between 25 and 30. "Obesity" is a condition in which the natural energy reserve, stored in the fatty tissue of animals, in particular humans and other mammals, is increased to a point where it is associated with certain health conditions or increased mortality. "Obese" may be defined for an adult human as having a BMI greater than 30 (World Health Organization, 'Obesity and overweight', Fact sheet No 311, updated Jan 2015). "Normal weight" for an adult human may be defined as a BMI of 18.5 to 25, whereas "underweight" may be defined as a BMI of less than 18.5.

In one embodiment the subject may have a BMI of 18 to 29. In one embodiment the subject may have a BMI of 25 or less. The subject may have a BMI of 18.5 to 25.

### OMEGA-3 POLYUNSATURATED FATTY ACID

Polyunsaturated fatty acids (PUFA) can be classified into two major families (depending on the position (n) of the first double bond nearest the methyl end of the fatty acid carbon chain. Thus, the omega-6 fatty acids have the first unsaturated double bond six carbon atoms from the omega (methyl) end of the molecule and additionally have a total of two or more double bonds, with each subsequent unsaturation occurring 3 additional carbon atoms toward the carboxyl end of the molecule. In contrast, the omega-3 fatty acids have the first unsaturated double bond three carbon atoms away from the omega end of the molecule and additionally have a total of three or more double bonds, with each subsequent unsaturation occurring 3 additional carbon atoms toward the carboxyl end of the molecule.

Table 4 summarizes the common names of omega-3 fatty acids and the abbreviations that will be used throughout the specification:

**Table 4**

| Common Name | Abbreviation | Shorthand notation |
|---|---|---|
| oleic acid | OA | 18:1^{Δ9} |
| Linoleic acid | LA | 18:2^{Δ9,12} |
| γ-Linolenic | GLA | 18:3^{Δ6,9,12} |
| di-homo γ-linolenic acid | DGLA | 20:3^{Δ8,11,14} |
| Arachidonic acid | ARA | 20:4^{Δ5,8,11,14} |
| α-linolenic acid | ALA | 18:3^{Δ9,12,15} |
| stearidonic acid | SDA | 18:4^{Δ6,9,12,15} |
| eicosatetraenoic acid | ETA | 20:4^{Δ8,11,14,17} |
| eicosapentaenoic acid | EPA | 20:5^{Δ5,8,11,14,17} |
| docosapentaenoic acid | DPA | 22:5^{Δ7,10,13,16,19} |
| docosahexaenoic acid | DHA | 22:6^{Δ4,7,10,13,16,19} |

The three main types of omega-3 PUFA involved in human physiology are α-linolenic acid (ALA) (found in plant oils), eicosapentaenoic acid (EPA), and docosahexaenoic acid (DHA) (both commonly found in marine oils). Currently the primary dietary source of omega-3 highly unsaturated fatty acids is from certain fish oils which can contain up to 20-30% of these fatty acids in their triacylglycerides. Consequently large quantities of fish oil are processed and encapsulated each year for sale as a dietary supplement. Common sources of plant oils containing ALA include walnut, edible seeds, clary sage seed oil, algal oil, flaxseed oil, Sacha Inchi oil, Echium oil, and hemp oil. Sources of EPA and DHA include fish oils, egg oil, squid oils, and krill oil.

The omega-3 PUFA may be provided in small amounts of oils containing high quantities of omega-3 PUFA, such as fish oils or microbial oils.

In one embodiment, the omega-3 PUFA is selected from EPA (C20:5, omega-3) and DHA (C22:6, omega-3) or a mixture of EPA and DHA.

The omega-3 PUFA, for example as provided in a nutritional composition as described herein, may comprise an EPA to DHA ratio of about 0.5:1 to 5:1, 1:1 to 3:1 or 1.5:1 to 2.5:1 wt/wt. The omega-3 PUFA may comprise an EPA to DHA ratio of about 0.5:1, 1:1, 1.5:1, 2:1, 2.5:1, 3:1 or 5:1 wt/wt. In one embodiment, the omega-3 PUFA comprises an EPA to DHA ratio of about 2.5:1 wt/wt.

An example of a commercial source of omega-3 PUFA is GNC Preventive Nutrition® Triple Strength Fish Oil.

### DOSAGE

The actual dosage of omega-3 PUFA may be varied to provide a dose which will be most suitable for an individual subject. The dose may vary with the age, weight and response of the particular subject.

The subject may, for example, be administered from about 500 to about 10000 mg omega-3 PUFA per day. In one embodiment the subject is administered from about 1500 to about 3000 mg omega-3 PUFA per day.

The subject may, for example, be administered about 500 to 4000 mg EPA per day. In one embodiment the subject is administered about 1000 to 3000 mg EPA per day. In one embodiment the subject is administered about 2000 mg EPA per day.

The subject may, for example, be administered about 500 to 4000 mg DHA per day. In one embodiment the subject is administered about 500 to 3000, 500 to 2000 or 500 to 1000 mg DHA per day. In one embodiment the subject is administered about 500 to 1000 mg DHA per day. In one embodiment the subject is administered about 750 mg DHA per day.

The subject may, for example, be administered about 500 to 4000 mg EPA and about 500 to 4000 mg DHA per day. In one embodiment the subject is administered about 1000 to 3000 mg EPA and about 500 to 1000 mg DHA per day. In one embodiment the subject is administered about 2000 mg EPA and about 750 mg DHA per day.

### NUTRITIONAL COMPOSITION

The term nutritional composition means a composition which nourishes a subject. The nutritional composition is usually to be taken orally, intragastrically or intravenously. Preferably, the nutritional composition for use in the present invention is to be taken orally. Nutritional compositions, as used herein, are understood to include any number of optional ingredients in addition to omega-3 PUFA. Such additional ingredients include, but are not limited to, conventional food additives (synthetic or natural), for example one or more acidulants, additional thickeners, buffers or agents for pH adjustment, chelating agents, colorants, emulsifies, excipient, flavor agent, mineral, osmotic agents, a pharmaceutically acceptable carrier, preservatives, stabilizers, sugar, sweeteners, texturizers, and/or vitamins. The optional ingredients can be added in any suitable amount.

The nutritional composition may be in the form of powder, tablets, capsules, pastilles or a liquid for example. The composition may further contain protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellifying agents and gel forming agents. The composition may also contain conventional pharmaceutical additives and adjuvants, excipients and diluents, including, but not limited to, water, gelatine of any origin, vegetable gums, lignin-sulfonate, talc, sugars, starch, gum arabic, vegetable oils, polyalkylene glycols, flavouring agents, preservatives, stabilizers, emulsifying agents, buffers, lubricants, colorants, wetting agents, fillers, and the like.

The composition may be usable for reconstitution in water. The composition may be an oil-in-water emulsion.

The nutritional composition may contain a carbohydrate source in addition to the omega-3 PUFA.

The nutritional composition may contain a source of lipids in addition to the omega-3 PUFA. Illustrative fat sources include palm oleic, high oleic sunflower oil and high oleic safflower oil. Other essential fatty acids, such as linoleic acid and arachidonic acid, may also be added.
In one embodiment, at least 70, 80, 90, 95, 98, 99 or 100% of the PUFA in the nutritional composition are omega-3 PUFA. In one embodiment the nutritional composition does not comprise omega-6 PUFA.

The nutritional composition may contain vitamins and minerals understood to be essential in the daily diet and in nutritionally significant amounts. Minimum requirements have been established for certain vitamins and minerals. Examples of minerals, vitamins and other nutrients optionally present in the composition include vitamin A, vitamin B1, vitamin B2, vitamin B6, vitamin B12, vitamin E, vitamin K, vitamin C, vitamin D, folic acid, inositol, niacin, biotin, pantothenic acid, choline, calcium, phosphorous, iodine, iron, magnesium, copper, zinc, manganese, chlorine, potassium, sodium, selenium, chromium, molybdenum, taurine, and L- carnitine. Minerals are usually added in salt form. The presence and amounts of specific minerals and other vitamins will vary depending on the intended population.

The nutritional composition may also contain other substances which may have a beneficial effect such as lactoferrin, nucleotides, nucleosides, gangliosides, polyamines, phytosterols, fibres, lipids and the like.

The nutritional composition may be in the form of a nutritional supplement. A nutritional supplement refers to a product which is intended to supplement the general diet of a subject.

The composition may be in the form of a complete nutritional product. A complete nutritional product refers to a product which is intended to be the sole item or meal or diet consumed by a subject. As such, a complete nutritional product may contain sufficient types and levels of macronutrients (proteins, fats and carbohydrates) to be sufficient to be a sole source of nutrition for the subject to which it is being administered.

The composition may be inserted or mixed into a food substance. The composition may be in the form of a food stuff, for example a human food stuff.

The nutritional composition may be in the form of a tablet or capsule.

The nutritional composition may be enriched with omega-3 PUFA. 'Enriched' means that omega-3 PUFA has been added to the composition. For example, omega-3 PUFA may be spiked (i.e. added within or into) the composition.

In one embodiment, where a nutritional composition natively contains omega-3 PUFA, enriched with omega-3 PUFA means that the enriched composition comprises a greater amount of the compound than occurs naturally natively in the composition.

For example, an enriched composition may comprise at least 1.5-, at least 2-, at least 5-, at least 10-, at least 20-, at least 50- or at least 100-fold more omega-3 PUFA than an equivalent naturally occurring native composition which has not been enriched.

Enrichment may be achieved by adding omega-3 PUFA to the composition, for example by spraying or spiking it with omega-3 PUFA.

### SUBJECT

The subject may be, but is not limited to, mammals such as bovine, canine, caprine, cervine, equine, feline, human, ovine, porcine and primates. The subject may be a companion animal. Preferably, the subject is a human. In various embodiments, the subject may have, or be suspected of or at risk of, a condition associated with elevated long-term glucose levels.

A condition associated with elevated long-term glucose levels includes insulin resistance, prediabetes, type 2 diabetes, metabolic syndrome or cardiovascular disease.

The subject may be at risk of developing, have a predisposition for or be suffering from elevated long-term glucose levels.

The subject may be at risk of developing, have a predisposition for or be suffering from insulin resistance, prediabetes or Type II Diabetes, metabolic syndrome or cardiovascular disease.

### USE

In one aspect, the present invention also provides the use of an omega-3 PUFA or a nutritional composition comprising omega-3 PUFA as an active agent for reducing long-term glucose levels in a subject.

The nutritional composition may be any nutritional composition as described herein.

### METHOD

In one aspect the present invention relates to a method for reducing long-term glucose levels in a subject comprising administering an omega-3 PUFA or a nutritional composition comprising omega-3 PUFA as an active agent to the subject.

In one aspect the present invention relates to a method for reducing long-term glucose levels to treat or prevent a condition associated with elevated long-term glucose levels in a subject comprising administering an omega-3 PUFA or a nutritional composition comprising omega-3 PUFA as an active agent to the subject.

The present methods comprise administering an effective amount of an omega-3 PUFA to the subject. An effective amount refers to an amount which is capable of, for example, reducing long-term glucose levels in the subject and/or preventing or a condition associated with elevated long-term glucose levels in the subject.

The effective amount may differ depending on the weight, age or sex of the subject.

Those skilled in the art will understand that they can freely combine all features of the present invention described herein, without departing from the scope of the invention as disclosed.

Various preferred features and embodiments of the present invention will now be described by way of non-limiting examples.

### EXAMPLES

### Example 1 - Impact of omega-3 PUFA supplementation on long-term glucose levels

The bioefficacy of omega-3 PUFA (provided by intake of fish oil supplements) on long-term glucose levels was assessed.

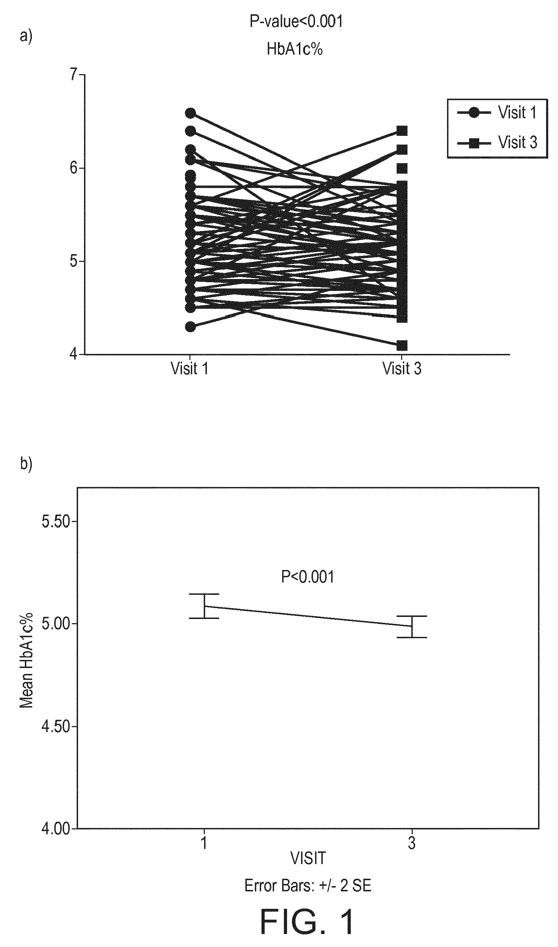

The present inventors determined that a six-week period of omega-3 supplementation reduced long-term glucose levels in subjects - as determined by measuring fasting HbA1c % (see Figure 1 and Table 1).

**Table 1 - Long-term glucose levels before and after omega-3 PUFA supplementation**

| Hb1Ac (%) | | Baseline | Week 6 | Change (Week 6-Baseline) | %Change ((Week 6/Baseline)-1)*100 |
|---|---|---|---|---|---|
| | **N** | **189** | **188** | **186** | **186** |
| | **Mean** | **5.139 (±0.348)** | **5.072 (±0.358)** | **-0.069 (±0.339)** | **-1.368** |
| | **Median** | **5.100** | **5.000** | **-0.100** | **-1.923** |

The results of the present study also demonstrated that the reduction in long-term glucose levels was independent of the subject's BMI. As such, the reduction in long-term glucose levels was achieved in both overweight subjects (i.e. with a BMI of more than 25) and subjects who were neither overweight nor obese (i.e. with a BMI of less than 25) (see Table 2).

**Table 2 a. and b. - Reduction in long-term glucose levels following omega-3 supplementation was independent of the subject's BMI.**

| a. | | | | | | |
|---|---|---|---|---|---|---|
| | | | **ANOVA^{a}** | | | |
| Model | | Sum of Squares | df | Mean Square | F | Sig. |
| | Regression | .078 | 1 | .078 | .110 | .740^{b} |
| 1 | Residual | 131.976 | 188 | .702 | | |
| | Total | 132.054 | 189 | | | |

| b. | | | | | | |
|---|---|---|---|---|---|---|
| | | **Coefficients^{a}** | | | | |
| Model | | Unstandardized Coefficients | | Standardized Coefficients | t | Sig. |
| | | B | Std. Error | Beta | | |
| 1 | (Constant) | -0.307 | 0.553 | | -0.556 | 0.579 |
| | BMI | 0.008 | 0.023 | 0.024 | 0.332 | 0.740 |

| | | | | | | |
|---|---|---|---|---|---|---|
| a. Dependent Variable: HbA1c& reduction at 6 weeks from baseline (visit 1) b. Predictors: (Constant), BMI at baseline (visit 1) a. Dependent Variable: HbA1c reduction at 6 weeks from baseline (visit 1) | | | | | | |

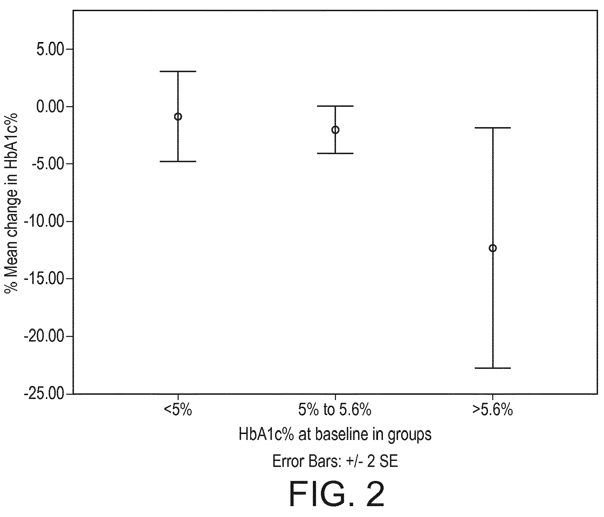

It was also determined that the decrease in Hb1Ac% was greatest in subjects with >5.6% Hb1Ac at baseline (i.e. including subjects with prediabetes) - see Figure 2.

### Materials and methods

### Study participants

191 healthy male and female subjects between 18 to 40 years of ages, with a BMI between 18.5 and 30, non-smokers, with sedentary to moderate physical activity and no consumption of dietary supplements or any medication that can affect the study outcome.

### Omega-3 PUFA dosage

The participants' diets were supplemented with omega-3 PUFA at a dose of 2700 mg day, as provided by three fish oil capsules (GNC Preventive Nutrition^{®} Triple Strength Fish Oil), to be orally administered each day. Each capsule contains 647 mg eicosapentaenoic acid (EPA) and 253 mg docosahexaenoic acid (DHA).

### Study procedure

Participants visited the clinical setting on three occasions, three weeks apart. After a screening interview, potential participants were invited to the study site for their first visit during which they provided informed consent. After verification of the inclusion/exclusion criteria, enrolled participants undertook the following tests: clinical history, food frequency questionnaire, validated physical activity questionnaire, blood pressure measurement, anthropometric measurements (weight, height and waist circumference), body composition by bioelectric impedance using the InBody720 equipment (Biospace Co, Ltd.). A 12.5 ml blood sample was drawn under fasting conditions (> 8 hrs) for measurements of HbA1c %, glucose, insulin, triglycerides, total cholesterol, lipoprotein fractions, adiponectin and CRP in plasma and separation of PBMC for RNA and DNA isolation. 2.5 ml EDTA-blood was drawn for determination of fatty acid in the plasma membrane of erythrocytes and 10 ml for PBMC transcriptomics. Participants were also provided with the supplements for the next three weeks.

During a second visit, the results of HbA1c %, glucose, lipids, insulin and body composition were discussed by a nutritionist with the subjects. The subjects returned a side effects journal, filled out a 24 hour food recall questionnaire and provided with the supplements for the last three weeks.

During the second visit, the participants discussed the results of the HbA1c%, glucose, lipids, insulin and body composition tests with a nutritionist. Participants also returned return the side effects journal, filled out a 24 hr food recall questionnaire and were provided with the supplements for the last three weeks. The last visit, after six weeks supplementation, had the same parameters as the first visit, except that the clinical history and SNUT food frequency questionnaire were recorded. The 24 hr food recall questionnaire and physical activity questionnaires were collected on all three visits.

All publications mentioned in the above specification are herein incorporated by reference. Various modifications and variations of the described methods of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in the art are intended to be within the scope of the following claims.

## Claims

1. A nutritional composition comprising an omega-3 PUFA as an active agent for use in reducing long-term glucose levels in a subject.

2. A nutritional composition according to claim 1 for use in reducing long-term glucose levels to treat or prevent a condition associated with elevated long-term glucose levels in a subject.

3. A nutritional composition for use according to claim 2 wherein the condition associated with elevated long-term glucose levels is insulin resistance, prediabetes, type 2 diabetes, metabolic syndrome or cardiovascular disease.

4. A nutritional composition for use according to any preceding claim wherein the reduction in long-term glucose levels is determined by reduced HbA1c levels.

5. A nutritional composition for use according to any preceding claim wherein the subject has a BMI of 25 or less.

6. A nutritional composition for use according to any preceding claim wherein the subject is administered between about 1000 and 5000 mg omega-3 PUFA per day, preferably between about 1500 and 3000 mg omega-3 PUFA per day.

7. A nutritional composition for use according to any preceding claim wherein the omega-3 PUFA is eicosapentanoic acid (EPA) and/or docosahexanoic acid (DHA).

8. A nutritional composition for use according to claim 7 wherein the omega-3 PUFA comprises an EPA to DHA ratio of about 0.5:1, 1:1, 1.5:1, 2:1, 2.5:1, 3:1 or 5:1 wt/wt; preferably about 2.5:1 wt/wt.

9. A nutritional composition for use according to claim 8 wherein the subject is administered about 500 to 4000 mg EPA and about 500 to 4000 mg DHA per day, preferably about 1000 to 3000 mg EPA and about 500 to 1000 mg DHA per day.

10. A nutritional composition for use according to any preceding claim wherein the composition is in the form of a foodstuff; preferably a human food stuff.

11. A nutritional composition for use according to any of claims 1 to 9 wherein the composition is in the form of a complete nutritional product.

12. A nutritional composition for use according to any of claims 1 to 9 wherein the composition is in the form of a capsule, a tablet, a powdered form, a freeze-dried product or an oil-in-water emulsion.

13. A method for reducing long-term glucose levels in a subject comprising administering a nutritional composition comprising omega-3 PUFA as an active agent to the subject.

14. A method according to claim 13 for use in treating or preventing a condition associated with elevated long-term glucose levels.

15. Use of an omega-3 PUFA in the preparation of a nutritional composition for reducing long-term glucose levels in a subject or for treating or preventing a condition associated with elevated long-term glucose levels in a subject.
